# EUROPEAN PATENT APPLICATION

(11) **EP 2 442 315 A2**
(43) Date of publication of application: **18.04.2012**
(21) Application number: 11183695.3
(22) Date of filing: 03.10.2011
(51) Int. Cl.: G21K 1/02, G01V 5/00, A61B 6/06

(54) **Hybrid collimator for X-rays and method of making same**

(30) Priority: 12.10.2010 US 902530
(71) Applicant: General Electric Company, Schenectady, NY 12345 (US)
(72) Inventor: Ikhlef, Abdelaziz, Waukesha, WI 53188 (US)
(74) Representative: Bedford, Grant Richard

(57) **Abstract**

An x-ray collimator (52) comprises a first plurality of x-ray attenuation plates (54) having a width (66) and a length (58), the length (58) extending along a first direction (56), wherein the plates (54) of the first plurality of x-ray attenuation plates (54) are spaced apart from one another along a second direction (62). The collimator comprises a second plurality of x-ray attenuation plates (60) having a width (68) and a length (64), the length (64) extending along the second direction (62), wherein the plates (60) of the second plurality of x-ray attenuation plates (60) are spaced apart from one another along the first direction (56) and wherein the plates (60) of the second plurality of x-ray attenuation plates (60) extend through the plates (54) of the first plurality of x-ray attenuation plates (54). The first and second directions (56,62) are orthogonal, and the width (66) of the plates (54) of the first plurality of x-ray attenuation plates (54) is greater than the width (68) of the plates (60) of the second plurality of x-ray attenuation plates (60).

## Description

Embodiments of the invention relate generally to diagnostic imaging and, more particularly, to a hybrid collimator for x-rays and a method of making same.

Typically, in computed tomography (CT) imaging systems, an x-ray source emits a fan-shaped beam toward a subject or object, such as a patient or a piece of luggage. Hereinafter, the terms "subject" and "object" shall include anything capable of being imaged. The beam, after being attenuated by the subject, impinges upon an array of radiation detectors. The intensity of the attenuated beam radiation received at the detector array is typically dependent upon the attenuation of the x-ray beam by the subject. Each detector element of the detector array produces a separate electrical signal indicative of the attenuated beam received by each detector element. The electrical signals are transmitted to a data processing system for analysis which ultimately produces an image.

Generally, the x-ray source and the detector array are rotated about the gantry within an imaging plane and around the subject. X-ray sources typically include x-ray tubes, which emit the x-ray beam at a focal point. X-ray detectors typically include a post patient x-ray collimator for collimating x-ray beams received at the detector, a scintillator adjacent to the collimator for converting x-rays to light energy, and photodiodes for receiving the light energy from the adjacent scintillator and producing electrical signals therefrom.

Typically, each scintillator of a scintillator array converts x-rays to light energy. Each scintillator discharges light energy to a photodiode adjacent thereto. Each photodiode detects the light energy and generates a corresponding electrical signal. The outputs of the photodiodes are then transmitted to the data processing system for image reconstruction.

The post patient x-ray collimator used in CT detection is a device mainly made of a highly absorbing material such as Tungsten or Molybdenum plates aligned to a focal spot on the x-ray tube. The main function of the collimator is to select x-rays along a particular direction (primary beam from focal spot) and to reject scattered radiation from other directions (patient scattered radiation). For this purpose, collimating plates are placed in front of the scintillator pixels to eliminate scattered radiation from the patient. In one example, a collimator includes tungsten plates placed in front of the interfaces of the detector cells (detector septa), requiring high precision manufacturing and alignment/precision features within the individual parts for alignment purpose. The dimension in the Y-axis (x-ray path/direction) is determined by the amount of the scatter-to-primary ratio (SPR) desired. Obviously, the higher the dimension in Y-axis, the lower is the SPR. Since the advent of multi-slice detectors, the coverage in Z-axis keeps increasing and leading to higher SPR. The high SPR has a significant impact in image quality in general and in low signal applications / Low Contrast Detectability Application (LCD) in particular.

Therefore, it would be desirable to design a collimator that reduces the scatter in two dimensions such that SPR is decreased.

According to an aspect of the invention, an x-ray collimator comprises a first plurality of x-ray attenuation plates having a width and a length, the length extending along a first direction, wherein the plates of the first plurality of x-ray attenuation plates are spaced apart from one another along a second direction. The collimator further comprises a second plurality of x-ray attenuation plates having a width and a length, the length extending along the second direction, wherein the plates of the second plurality of x-ray attenuation plates are spaced apart from one another along the first direction and wherein the plates of the second plurality of x-ray attenuation plates extend through the plates of the first plurality of x-ray attenuation plates. The first and second directions are orthogonal, and the width of the plates of the first plurality of x-ray attenuation plates is greater than the width of the plates of the second plurality of x-ray attenuation plates.

According to another aspect of the invention, a CT system comprises a rotatable gantry having an opening to receive an object to be scanned, an x-ray projection source positioned on the rotatable gantry and configured to project a beam of x-rays from a focal spot of the x-ray projection source toward the object, and a detector module positioned on the rotatable gantry. The detector comprises a two-dimensional array of detector cells configured to receive x-rays attenuated by the object, wherein a space between neighboring detector cells forms a two-dimensional grid of septa having a first plurality of septa aligned in parallel along a first dimension and a second plurality of septa aligned in parallel along a second dimension orthogonal to the first dimension and a collimator positioned on the rotatable gantry adjacently to the detector module. The collimator is configured to collimate x-rays impinging thereon and comprises a first plurality of plates aligned with the first plurality of septa and a second plurality of plates aligned with the second plurality of septa; the second plurality of plates having a portion thereof extending through the first plurality of plates. A width of the second plurality of plates along a third dimension orthogonal to the first and second dimensions is less that a width of the first plurality of plates along a third dimension.

According to yet another aspect of the invention, a method of making an x-ray collimator comprises forming a plurality of slots along a length of a first plurality of x-ray attenuation plates, each slot extending along a width of a respective x-ray attenuation plate and aligning the lengths of the first plurality of x-ray attenuation plates along a first direction such that a each slot in one of the first plurality of x-ray attenuation plates is aligned with a corresponding slot in each of the other first plurality of x-ray attenuation plates along a second direction orthogonal to the first direction to form a plurality of aligned slots. The method also comprises inserting each x-ray attenuation plate of a second plurality of x-ray attenuation plates through a respective aligned slot of the plurality of aligned slots such that a length of the each x-ray attenuation plate is aligned along the second direction and wherein a width of the x-ray attenuation plates of the second plurality of x-ray attenuation plates is less than the width of the x-ray attenuation plates of the first plurality of x-ray attenuation plates.

Various other features and advantages will be made apparent from the following detailed description and the drawings.

The drawings illustrate preferred embodiments presently contemplated for carrying out the invention.

In the drawings:
FIG. 1 is a pictorial view of a CT imaging system.
FIG. 2 is a block schematic diagram of the system illustrated in FIG. 1.
FIG. 3 is an isometric view of an x-ray collimator according to an embodiment of the invention.
FIG. 4 is a schematic diagram illustrating the collimator of FIG. 3 positioned adjacently to a detector array according to an embodiment of the invention.
FIG. 5 is an exploded isometric view of line 5-5 of FIG. 4.
FIG. 6 is an isometric view of a plurality of plates of the collimator of FIG. 3 according to an embodiment of the invention.
FIG. 7 is a schematic view of a plate of the collimator of FIG. 3 according to an embodiment of the invention.
FIG. 8 is an isometric view of a plurality of plates of the collimator of FIG. 3 according to an embodiment of the invention.
FIG. 9 is a schematic view of a plate of the collimator of FIG. 3 according to an embodiment of the invention.
FIG. 10 is a schematic view of a plate of the collimator of FIG. 3 according to an embodiment of the invention.
FIG. 11 is a schematic view of a plate of the collimator of FIG. 3 according to an embodiment of the invention.
FIG. 12 is a pictorial view of a CT system for use with a non-invasive package inspection system.

The operating environment of various embodiments of the invention is described with respect to a 256-slice computed tomography (CT) system. However, it will be appreciated by those skilled in the art that aspects of the invention are equally applicable for use with other multi-slice configurations. Moreover, embodiments of the invention will be described with respect to the detection and conversion of x-rays. However, one skilled in the art will further appreciate that aspects of the invention are equally applicable for the detection and conversion of other high frequency electromagnetic energy. Embodiments of the invention will be described with respect to a "third generation" CT scanner, but are equally applicable with other CT systems.

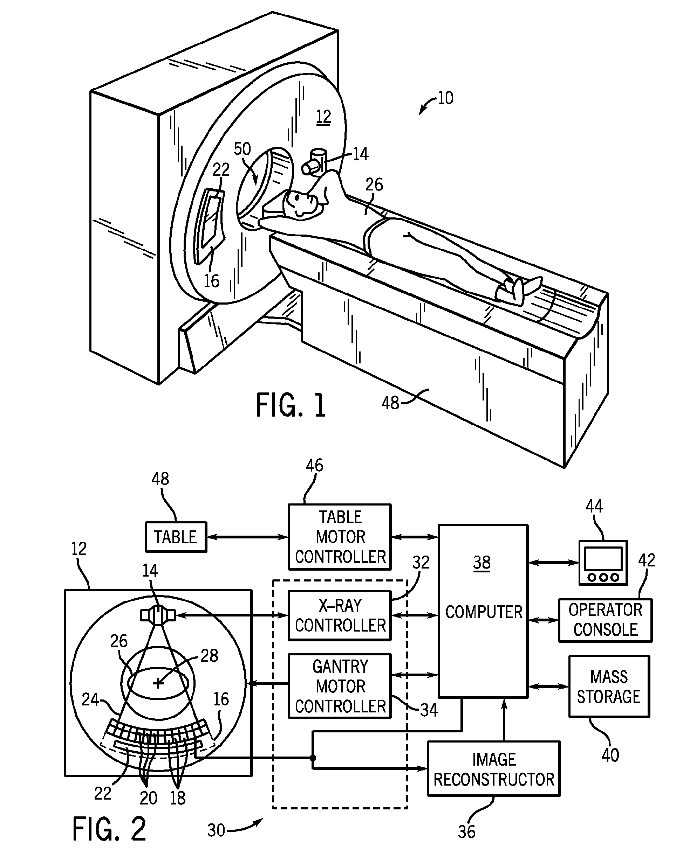

Referring to FIG. 1, a computed tomography (CT) imaging system 10 is shown as including a gantry 12 representative of a "third generation" CT scanner. Gantry 12 has an x-ray source 14 that projects a beam of x-rays toward a detector assembly 16 on the opposite side of the gantry 12. Referring now to FIG. 2, detector assembly 16 is formed by a plurality of detectors 18, a plurality of collimator assemblies 20, and a data acquisition system (DAS) 22. The plurality of detectors 18 sense the projected x-rays 24 that pass through a medical patient 26, and DAS 22 converts the data to digital signals for subsequent processing. Each detector 18 produces an analog electrical signal that represents the intensity of an impinging x-ray beam and hence the attenuated beam as it passes through the patient 26. During a scan to acquire x-ray projection data, gantry 12 and the components mounted thereon rotate about a center of rotation 28.

Rotation of gantry 12 and the operation of x-ray source 14 are governed by a control mechanism 30 of CT system 10. Control mechanism 30 includes an x-ray controller 32 that provides power and timing signals to an x-ray source 14 and a gantry motor controller 34 that controls the rotational speed and position of gantry 12. An image reconstructor 36 receives sampled and digitized x-ray data from DAS 22 and performs high speed reconstruction. The reconstructed image is applied as an input to a computer 38 which stores the image in a mass storage device 40.

Computer 38 also receives commands and scanning parameters from an operator via console 42 that has some form of operator interface, such as a keyboard, mouse, voice activated controller, or any other suitable input apparatus. An associated display 44 allows the operator to observe the reconstructed image and other data from computer 38. The operator supplied commands and parameters are used by computer 38 to provide control signals and information to DAS 22, x-ray controller 32 and gantry motor controller 34. In addition, computer 38 operates a table motor controller 46 which controls a motorized table 48 to position patient 26 and gantry 12. Particularly, table 48 moves patients 26 through a gantry opening 50 of FIG. 1 in whole or in part.

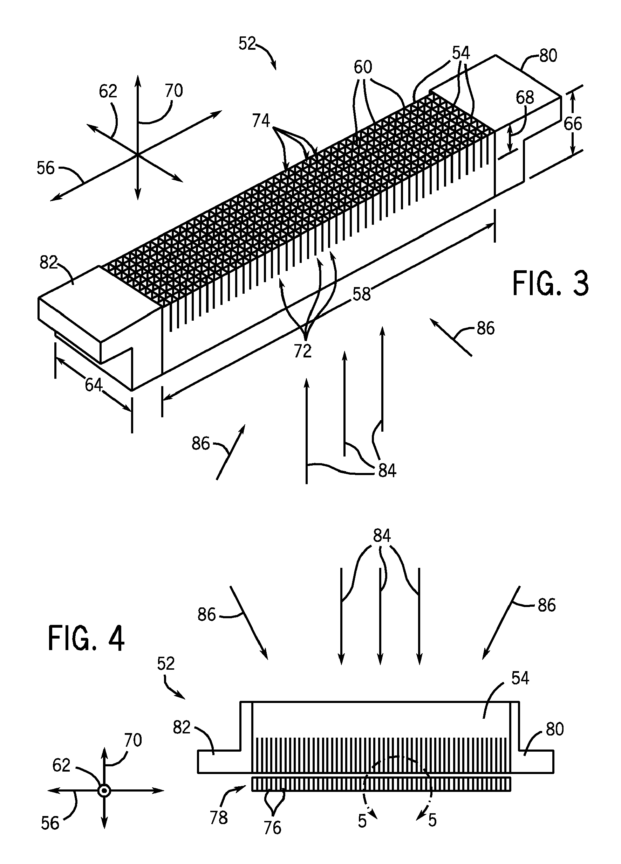

FIG. 3 is an isometric view of an x-ray collimator 52 of a collimator assembly 20 of FIG. 2 according to an embodiment of the invention. Collimator 52 includes a plurality of plates 54 positioned along a first axis 56 such that a length 58 of the plates 54 lies along the first axis 56. Collimator 52 includes a plurality of plates 60 positioned along a second axis 62 such that a length 64 of the plates 60 lies along the second axis 62 that is orthogonal to first axis 56. In one embodiment, first axis 56 corresponds with a slice direction, and second axis 62 corresponds with a channel direction. When positioned in this manner, plates 54 are aligned in the direction along first axis 56 while being separated from each other in the second axis 62 direction, and plates 60 are aligned in the direction along second axis 62 while being separated from each other in the first axis 56 direction.

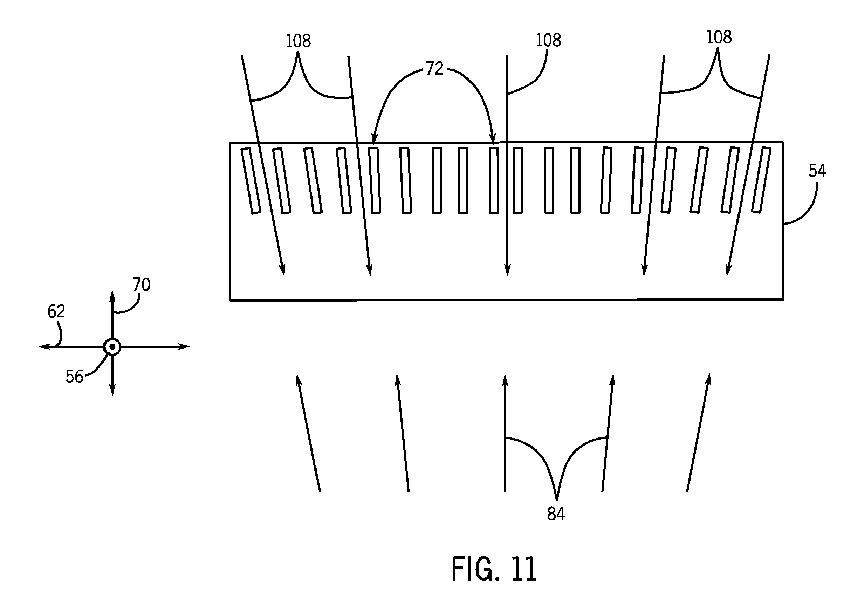

A width 66 of plates 54 and a width 68 of plates 60 generally extend along a third axis 70 orthogonal to both the first and second axes 56, 62. In one embodiment, plates 54, 60 may not all be parallel with each other when a focusing arrangement of plates 54 and/or plates 60 toward an x-ray source, such as x-ray source 14, is desired as discussed below with respect to FIG. 11. Even when a focusing arrangement is designed, the widths 66, 68 of plates 54, 60 extend along respective directions more closely aligned with third axis 70 than with either first or second axis 56, 62.

As shown and discussed below, plates 60 are coupled to plates 54 via a plurality of slots 72 formed in each plate 54. Referring to FIGS. 3 and 4, the grid of interlocking plates 54, 60 forms a plurality of collimating passageways or volumes 74 and is designed to correspond, in one embodiment, with the number of detector cells 76 of a detector array 78 for which x-ray collimation is desired. That is, each plate 54 is separated from a neighboring plate 54 by a distance corresponding to, for example, a channel width of detector array 78, and each plate 60 is separated from a neighboring plate 60 by a distance corresponding to, for example, a slice width of detector array 78. According to embodiments of the invention, collimator 52 may be designed to have up to two-hundred and fifty-six collimating passageways 74 or more in the slice direction and up to sixty-four collimating passageways 74 or more in the slice direction.

Collimator 52 includes a pair of mounting members 80, 82 configured to couple collimator 52 to a plurality of rails (not shown) of a detector assembly (not shown). In this manner, a plurality of collimators 52 may be positioned adjacently to one another to create a collimator assembly having, for example, 256 rows and 912 columns.

Widths 66, 68 of plates 54, 60 are designed to allow primary x-rays 84 from an x-ray source, such as x-ray source 14, to pass through collimating passageways 74 to impinge on detector cells 76 and to fully attenuate or absorb scattered x-rays 86 such that scattered x-rays 86 are prevented from impinging on detector cells 76. Plates 54 are constructed of an x-ray attenuation material such as tungsten, molybdenum, tantalum, high z-material alloys, or the like. Plates 60 are also constructed of an x-ray attenuation material such as tungsten, molybdenum, tantalum, high z-material alloys, or the like but need not be constructed of the same material as plates 54.

According to another embodiment of the invention, the number of collimating passageways 74 in collimator 52 is less than the number of detector cells 76. For example, each collimating passageway 74 may correspond with two detector cells 76 such that a plate 60 is positioned in collimator 52 to correspond with every other detector cells 76. Depending on the scatter-to-primary ratio (SPR) desired, plates 60 may be positioned such that each collimating passageway 74 corresponds with two, three, or more detector cells 76. In addition, plates 54 may also be positioned such that collimating passageways 74 correspond with one- or two-dimensional groups of detector cells 76.

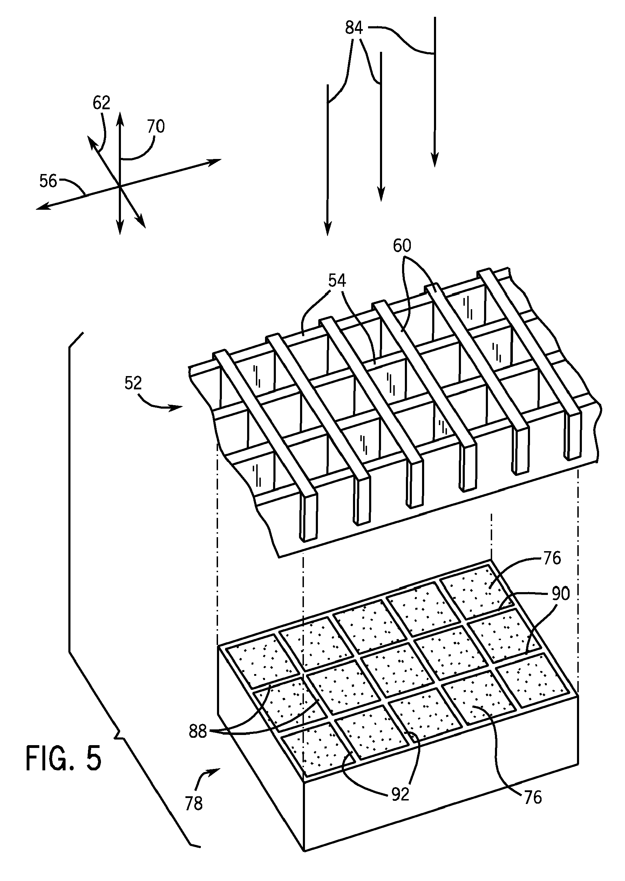

FIG. 5 illustrates an exploded isometric view of the collimator and detector assembly of FIG. 4 about line 5-5. As shown detector array 78 includes a two-dimensional array of detector cells 76. A two-dimensional grid of septa 88 is formed between each neighboring pair of detector cells 76. Plates 54 are positioned to correspond with a first subset of septa 90 oriented in a direction corresponding with first axis 56, and plates 60 are positioned to correspond with a second subset of septa 92 oriented in a direction corresponding with second axis 62. In one embodiment, a thickness of plates 54 in the direction corresponding with second axis 62 and the thickness of plates 60 in the direction corresponding with first axis 56 are greater than the thicknesses corresponding to septa 90, 92. In this manner, collimator 52 is configured to absorb primary x-rays 84 that would otherwise impinge on septa 90, 92.

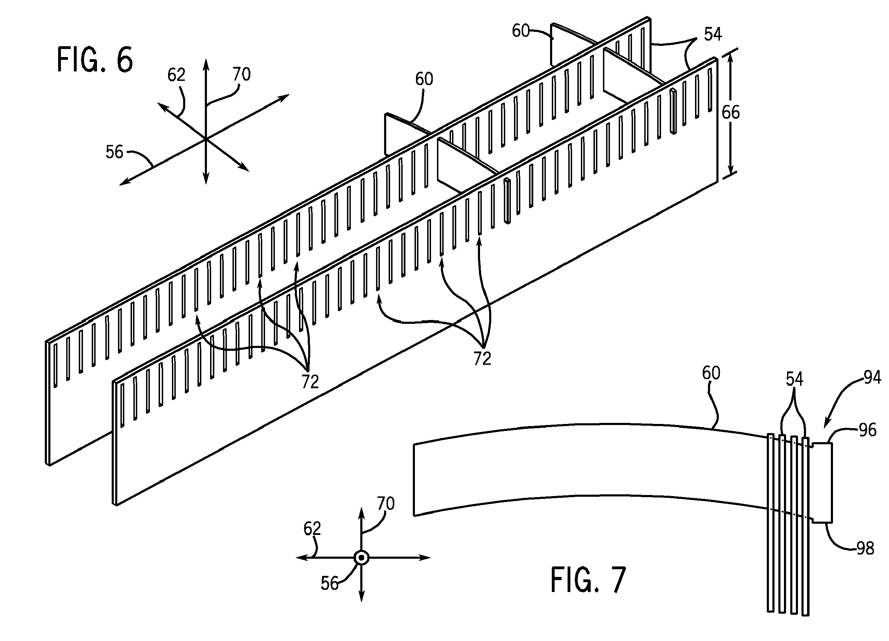

FIG. 6 shows an isometric view of a pair of plates 54 and a pair of plates 60 to illustrate a relation of the plates 54, 60 of collimator 52 of FIG. 3 according to an embodiment of the invention. As shown, slots 72 are formed in each plate 54 such that plate 60 may be positioned to extend through each plate 54. As shown in FIG. 6, slots 72 are formed to extend in a direction generally aligned with width 66. Slots 72 are closed such that the material of the respective plate 54 surrounds each slot 72 in a plane formed by first axis 56 and third axis 70. When the plates 54 are aligned, each slot 72 in one plate 54 is aligned with a corresponding slot 72 in each of the other plates 54 such that a plurality of aligned slots 72 in the direction corresponding with second axis 62 is formed. To couple plates 60 to plates 54, plates 60 are inserted through respective aligned slots 72 in a direction substantially aligned with second axis 62. Plates 54, plates 60 are locked to each other using high reliable adhesive.

As illustrated in FIG. 6, plates 60 have a substantially rectangular profile. According to another embodiment as illustrated in FIG. 7, a portion of the profile of plates 60 may be curved. The amount of curvature of plates 60 corresponds, in one embodiment, with the curvature of detector assembly 16 shown in FIG. 1.

A plurality of plates 54 is shown in phantom to illustrate an engagement of plates 54 with plates 60. A first end 94 of plate 60 may be formed to have one or more tabs 96, 98 to help prevent first end 94 of plate 60 from passing through plates 54 for manufacturability purpose.

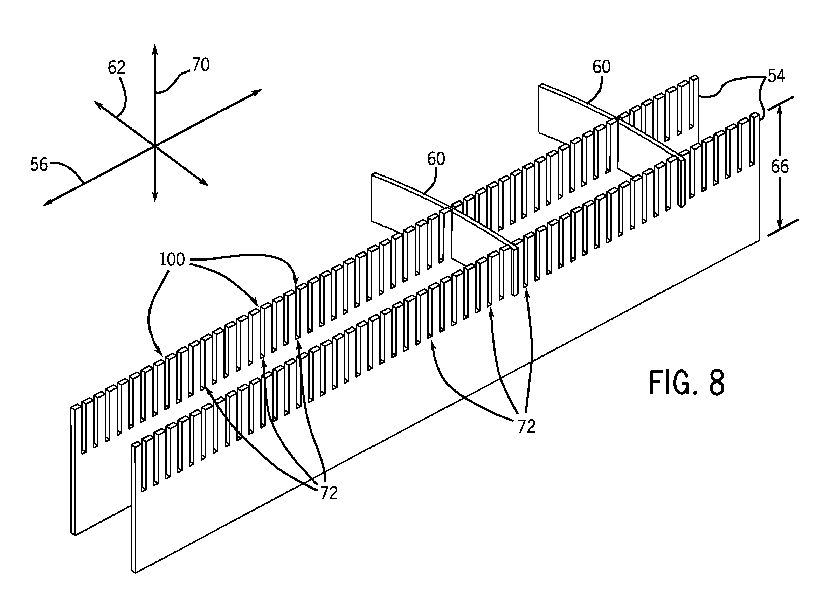

FIG. 8 shows an isometric view of a pair of plates 54 and a pair of plates 60 to illustrate a relation of the plates 54, 60 of collimator 52 according to an embodiment of the invention. As shown, slots 72 are formed in each plate 54 such that plate 60 may be positioned to extend through each plate 54. As shown in FIG. 6, slots 72 are formed to extend in a direction generally aligned with width 66. Slots 72 are open such that the material of the respective plate 54 fails to completely surround each slot 72 in a plane formed by first axis 56 and third axis 70 such that an opening 100 is formed. To couple plates 60 to plates 54, plates 60 may be inserted through slots 72 in a direction substantially aligned with second axis 62 or in a direction substantially aligned with third axis 70. Here also, both types of plates are locked to each other using high reliable adhesive.

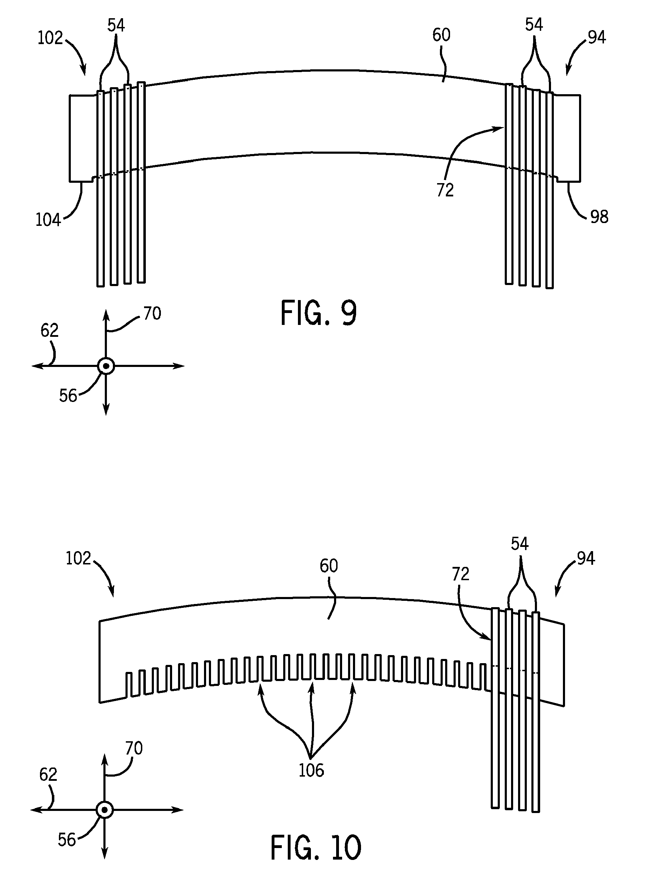

FIG. 9 shows a plate 60 of FIG. 8 according to an embodiment of the invention. As shown, a portion of the profile of plate 60 is curved, and as described above, the curvature may correspond, in one embodiment, with the curvature of detector assembly 16 shown in FIG. 1. Similar to the plate 60 shown in FIG. 7, the plate 60 shown in FIG. 9 may have tab 98 formed at first end 94 to help prevent first end 94 from sliding through plates 54. In addition, a second end 102 of plate 60 may be formed to have a tab 104 to help prevent second end 102 of plate 60 from passing through plates 54.

FIG. 10 illustrates an embodiment of a plate 60 configured to engage plates 54 of FIG. 8 according to another embodiment of the invention. Plate 60 has a plurality of open slots 106 formed therein configured to engage plates 54. Slots 106 are open such that the material of the respective plate 60 fails to completely surround each slot 106 in a plane formed by second axis 62 and third axis 70. Plate 60 is inserted into and extends through a slot 72 of a respective plate 54 such that the respective plate 54 is also inserted into and extends through a respective slot 106. An engagement of plate 60 with plates 54 in this manner mutually locks plates 54, 60 together such that the movement of plate 60 separate from plates 54 in the direction substantially corresponding with second axis 62 is avoided. In addition, the interlocking of plates 54, 60 would also eliminate the need to form tabs at either first or second end 94, 102 of plate 60 as discussed above.

FIG. 11 is a schematic view of a plate 54 illustrating an arrangement of forming slots 72 such that the collimating passageways 74 of collimator 52 have a focus 108 directed toward a focal point (not shown) of the source (not shown) of primary x-rays 84.

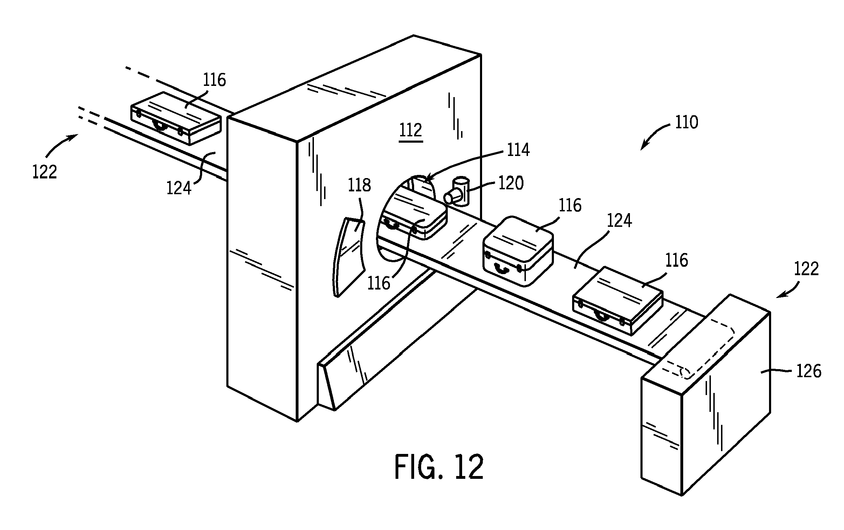

Referring now to FIG. 12, is a pictorial view of an x-ray imaging system 110 for use with a non-invasive package inspection system. The x-ray system includes 110 a gantry 112 having an opening 114 therein through which a plurality of packages or pieces of baggage 116 may pass. The gantry 112 houses a detector assembly 118 and a high frequency electromagnetic energy source, such as an x-ray tube 120. A conveyor system 122 is also provided and includes a conveyor belt 124 supported by a structure 126 to automatically and continuously pass packages or baggage pieces 116 through opening 114 to be scanned. Obj ects 116 are fed through opening 114 by conveyor belt 124, imaging data is then acquired, and the conveyor belt 124 removes the packages 116 from opening 114 in a controlled and continuous manner. As a result, postal inspectors, baggage handlers, and other security personnel may non-invasively inspect the contents of packages 116 for explosives, knives, guns, contraband, etc. One skilled in the art will recognize that gantry 112 may be stationary or rotatable. In the case of a rotatable gantry 112, system 110 may be configured to operate as a CT system for baggage scanning or other industrial or medical applications.

A collimator according to embodiments of the invention allows for high scatter rejection (e.g., an SPR less than 10% may be achieved) for 160 mm X-ray coverage at ISO (corresponding to a size of imaging an organ such as the heart) and includes a high stiffness up to high G-loads. Accordingly, speed calibrations become more simplified. In addition, a hybrid 1D-2D collimator as described herein has an exterior envelope that is interchangeable with existing 1D collimators and allows for the upgrade of such 1D collimators.

Therefore, according to an embodiment of the invention, an x-ray collimator comprises a first plurality of x-ray attenuation plates having a width and a length, the length extending along a first direction, wherein the plates of the first plurality of x-ray attenuation plates are spaced apart from one another along a second direction. The collimator further comprises a second plurality of x-ray attenuation plates having a width and a length, the length extending along the second direction, wherein the plates of the second plurality of x-ray attenuation plates are spaced apart from one another along the first direction and wherein the plates of the second plurality of x-ray attenuation plates extend through the plates of the first plurality of x-ray attenuation plates. The first and second directions are orthogonal, and the width of the plates of the first plurality of x-ray attenuation plates is greater than the width of the plates of the second plurality of x-ray attenuation plates.

According to another embodiment of the invention, a CT system comprises a rotatable gantry having an opening to receive an object to be scanned, an x-ray projection source positioned on the rotatable gantry and configured to project a beam of x-rays from a focal spot of the x-ray projection source toward the object, and a detector module positioned on the rotatable gantry. The detector comprises a two-dimensional array of detector cells configured to receive x-rays attenuated by the object, wherein a space between neighboring detector cells forms a two-dimensional grid of septa having a first plurality of septa aligned in parallel along a first dimension and a second plurality of septa aligned in parallel along a second dimension orthogonal to the first dimension and a collimator positioned on the rotatable gantry adjacently to the detector module. The collimator is configured to collimate x-rays impinging thereon and comprises a first plurality of plates aligned with the first plurality of septa and a second plurality of plates aligned with the second plurality of septa; the second plurality of plates having a portion thereof extending through the first plurality of plates. A width of the second plurality of plates along a third dimension orthogonal to the first and second dimensions is less that a width of the first plurality of plates along a third dimension.

According to yet another embodiment of the invention, a method of making an x-ray collimator comprises forming a plurality of slots along a length of a first plurality of x-ray attenuation plates, each slot extending along a width of a respective x-ray attenuation plate and aligning the lengths of the first plurality of x-ray attenuation plates along a first direction such that a each slot in one of the first plurality of x-ray attenuation plates is aligned with a corresponding slot in each of the other first plurality of x-ray attenuation plates along a second direction orthogonal to the first direction to form a plurality of aligned slots. The method also comprises inserting each x-ray attenuation plate of a second plurality of x-ray attenuation plates through a respective aligned slot of the plurality of aligned slots such that a length of the each x-ray attenuation plate is aligned along the second direction and wherein a width of the x-ray attenuation plates of the second plurality of x-ray attenuation plates is less than the width of the x-ray attenuation plates of the first plurality of x-ray attenuation plates.

This written description uses examples to disclose the invention, including the preferred mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. An x-ray collimator comprising:
   a first plurality of x-ray attenuation plates having a width and a length, the length extending along a first direction, wherein the plates of the first plurality of x-ray attenuation plates are spaced apart from one another along a second direction;
   a second plurality of x-ray attenuation plates having a width and a length, the length extending along the second direction, wherein the plates of the second plurality of x-ray attenuation plates are spaced apart from one another along the first direction and wherein the plates of the second plurality of x-ray attenuation plates extend through the plates of the first plurality of x-ray attenuation plates;
   wherein the first and second directions are orthogonal; and
   wherein the width of the plates of the first plurality of x-ray attenuation plates is greater than the width of the plates of the second plurality of x-ray attenuation plates.
2. The x-ray collimator of clause 1, wherein the first direction corresponds to a slice direction.
3. The x-ray collimator of any preceding clause, wherein the second direction corresponds to a channel direction.
4. The x-ray collimator of any preceding clause, wherein the plates of the second plurality of x-ray attenuation plates are positioned such that a passage formed between each pair of neighboring plates is focused toward a focal point.
5. The x-ray collimator of any preceding clause, wherein each plate of the first plurality of x-ray attenuation plates comprises a plurality of slots formed therethrough, wherein each slot has to a respective plate of the second plurality of x-ray attenuation plates positioned therein.
6. The x-ray collimator of any preceding clause, wherein each slot is surrounded by a material of the plate having the slot formed therethrough.
7. The x-ray collimator of any preceding clause, wherein a perimeter of each slot has an opening such that a material of the plate having the slot formed therethrough fails to surround the slot.
8. The x-ray collimator of any preceding clause, wherein each plate of the second plurality of x-ray attenuation plates comprises a plurality of slots formed therethrough;
   wherein a perimeter of each slot of the second plurality of x-ray attenuation plates has an opening such that a material of the plate having the slot formed therethrough fails to surround the slot; and
   wherein each slot of the second plurality of x-ray attenuation plates has a respective plate of the first plurality of x-ray attenuation plates positioned therein.
9. The x-ray collimator of any preceding clause, wherein a portion of a profile of the x-ray attenuation plates of the second plurality of x-ray attenuation plates is curved.
10. The x-ray collimator of any preceding clause, wherein the plates of the first plurality of x-ray attenuation plates are configured to fully attenuate x-rays impinging thereon and are constructed of a material comprising one of tungsten, molybdenum, tantalum, and a high z-material alloy.
11. The x-ray collimator of any preceding clause, wherein the plates of the second plurality of x-ray attenuation plates are configured to fully attenuate x-rays impinging thereon and are constructed of a different material than the material of the plates of the first plurality of x-ray attenuation plates.
12. A CT system comprising:
   a rotatable gantry having an opening to receive an object to be scanned;
   an x-ray projection source positioned on the rotatable gantry and configured to project a beam of x-rays from a focal spot of the x-ray projection source toward the object;
   a detector module positioned on the rotatable gantry and comprising:
      a two-dimensional array of detector cells configured to receive x-rays attenuated by the object, wherein a space between neighboring detector cells forms a two-dimensional grid of septa having a first plurality of septa aligned in parallel along a first dimension and a second plurality of septa aligned in parallel along a second dimension orthogonal to the first dimension; and
      a collimator positioned on the rotatable gantry adjacently to the detector module, the collimator configured to collimate x-rays impinging thereon and comprising:
         a first plurality of plates aligned with the first plurality of septa;
         a second plurality of plates aligned with the second plurality of septa; the second plurality of plates having a portion thereof extending through the first plurality of plates; and
         wherein a width of the second plurality of plates along a third dimension orthogonal to the first and second dimensions is less that a width of the first plurality of plates along a third dimension.
13. The CT system of any preceding clause, wherein the plates of the first and second plurality of plates are aligned to prevent impingement of x-rays on the respective septa and to prevent impingement of scattered x-rays on the detector cells.
14. The CT system of any preceding clause, wherein the plates of the second plurality of plates are focused toward the focal spot.
15. The CT system of any preceding clause, wherein each plate of the second plurality of plates extends through a slot in each plate of the first plurality of plates.
16. The CT system of any preceding clause, wherein each plate of the second plurality of plates has a slot formed therein aligned with the slot in each plate of the first plurality of plates.
17. The CT system of any preceding clause, wherein the plates of the first and second plurality of x-ray attenuation plates are constructed of a material comprising one of tungsten, molybdenum, tantalum, and a high z-material alloy.
18. A method of making an x-ray collimator comprising:
   forming a plurality of slots along a length of a first plurality of x-ray attenuation plates, each slot extending along a width of a respective x-ray attenuation plate;
   aligning the lengths of the first plurality of x-ray attenuation plates along a first direction such that a each slot in one of the first plurality of x-ray attenuation plates is aligned with a corresponding slot in each of the other first plurality of x-ray attenuation plates along a second direction orthogonal to the first direction to form a plurality of aligned slots;
   inserting each x-ray attenuation plate of a second plurality of x-ray attenuation plates through a respective aligned slot of the plurality of aligned slots such that a length of the each x-ray attenuation plate is aligned along the second direction; and
   wherein a width of the x-ray attenuation plates of the second plurality of x-ray attenuation plates is less than the width of the x-ray attenuation plates of the first plurality of x-ray attenuation plates.
19. The method of any preceding clause, wherein forming the plurality of slots comprises forming the plurality of slots such that a focus of the second plurality of x-ray attenuation plates is directed toward a common focal spot.
20. The method of any preceding clause, wherein aligning the lengths of the first plurality of x-ray attenuation plates comprises aligning the first plurality of x-ray attenuation plates such that a spacing between neighboring plates of the first plurality of x-ray attenuation plates corresponds with a first detector cell dimension of a detector array; and
   wherein forming the plurality of slots comprises forming the plurality of slots such that a spacing between neighboring plates of the second plurality of x-ray attenuation plates corresponds with a second detector cell dimension of the detector array.

## Claims

1. An x-ray collimator (52) comprising:
a first plurality of x-ray attenuation plates (54) having a width (66) and a length (58), the length (58) extending along a first direction (56), wherein the plates (54) of the first plurality of x-ray attenuation plates (54) are spaced apart from one another along a second direction (62);
a second plurality of x-ray attenuation plates (60) having a width (68) and a length (64), the length (64) extending along the second direction (62), wherein the plates (60) of the second plurality of x-ray attenuation plates (60) are spaced apart from one another along the first direction (56) and wherein the plates (60) of the second plurality of x-ray attenuation plates (60) extend through the plates (54) of the first plurality of x-ray attenuation plates (54);
wherein the first and second directions (56,62) are orthogonal; and
wherein the width (66) of the plates (54) of the first plurality of x-ray attenuation plates (54) is greater than the width (68) of the plates (60) of the second plurality of x-ray attenuation plates (60).

2. The x-ray collimator (52) of claim 1, wherein the first direction corresponds (56) to a slice direction.

3. The x-ray collimator (52) of any preceding claim, wherein the second direction (62) corresponds to a channel direction.

4. The x-ray collimator (52) of any preceding claim, wherein the plates (60) of the second plurality of x-ray attenuation plates (60) are positioned such that a passage (74) formed between each pair of neighboring plates (60) is focused toward a focal point.

5. The x-ray collimator (52) of any preceding claim, wherein each plate (54) of the first plurality of x-ray attenuation plates (54) comprises a plurality of slots (72) formed therethrough, wherein each slot (72) has to a respective plate (60) of the second plurality of x-ray attenuation plates (60) positioned therein.

6. The x-ray collimator (52) of claim 5, wherein each slot (72) is surrounded by a material of the plate (54) having the slot formed therethrough.

7. The x-ray collimator (52) of claim 5 or claim 6, wherein a perimeter of each slot (72) has an opening such that a material of the plate (54) having the slot (72) formed therethrough fails to surround the slot (72).

8. The x-ray collimator (52) of any preceding claim, wherein each plate (60) of the second plurality of x-ray attenuation plates (60) comprises a plurality of slots (106) formed therethrough;
wherein a perimeter of each slot (106) of the second plurality of x-ray attenuation plates (60) has an opening such that a material of the plate (60) having the slot (106) formed therethrough fails to surround the slot (106); and
wherein each slot (106) of the second plurality of x-ray attenuation plates (60) has a respective plate (54) of the first plurality of x-ray attenuation plates (54) positioned therein.

9. The x-ray collimator (52) of any preceding claim, wherein a portion of a profile of the x-ray attenuation plates (60) of the second plurality of x-ray attenuation plates (60) is curved.

10. The x-ray collimator (52) of any preceding claim, wherein the plates (54) of the first plurality of x-ray attenuation plates (54) are configured to fully attenuate x-rays impinging thereon and are constructed of a material comprising one of tungsten, molybdenum, tantalum, and a high z-material alloy.
